# EUROPEAN PATENT SPECIFICATION

(11) **EP 4 366 658 B1**
(45) Date of publication and mention of the grant of the patent: **19.02.2025**
(21) Application number: 22803088.8
(22) Date of filing: 08.07.2022
(51) Int. Cl.: A61F 2/00

(54) **AN IMPROVED PUMP FOR AN ARTIFICIAL SPHINCHTER**
VERBESSERTE PUMPE FÜR EINEN KÜNSTLICHEN SPHINKTER
POMPE AMÉLIORÉE POUR SPHINCTER ARTIFICIEL

(30) Priority: 08.07.2021 WO PCT/TR2021/050700
(43) Date of publication of application: 15.05.2024
(73) Proprietor: Lüleci, Hüseyin, 34805 Beykoz/Istanbul (TR)
(72) Inventor: LÜLECI, Ahmet Melih, 34805 Beykoz/Istanbul (TR); LÜLECI, Hüseyin, 34805 Beykoz/Istanbul (TR)
(74) Representative: Genç Ilhan, Oznur
(86) International application number: PCT/TR2022/050738
(87) International publication number: WO 2023/282883

(56) References cited:
- WO-A1-2018/156092
- US-A- 4 222 377
- US-A1- 2003 024 571

## Description

### TECHNICAL FIELD OF THE INVENTION

The present invention generally relates to an apparatus for treating incontinence and more specifically relates an apparatus for providing an inflatable artificial sphincter for control of excretory body passages. The invention provides a novel solution which effectively occludes excretory body passage of a patient even when a sudden pressure increment occurs in the abdomen of a patient to which an artificial sphincter is implanted.

### BACKGROUND OF THE INVENTION

A biological urinary sphincter prevents urinary flow via mucosal coaptation, compression and pressure transmission. On the other hand, an artificial urinary sphincter mimics the biological urinary sphincter by providing a competent bladder outlet during urinary storage and an open unobstructed outlet to permit voluntary urination. Similarly, an artificial rectal sphincter may be used to treat fecal incontinence caused by neurological or muscular dysfunction of an anal sphincter.

A known treatment for some cases of incontinence is to provide a patient with a mechanism to occlude the affected excretory body passage. These mechanisms are typically surgically implanted within the patient's body and are adapted to be operable by the patient to selectively open and occlude the body passage. Inflatable artificial sphincters are well known devices in the state of the art. Inflatable sphincters typically include an inflatable cuff for surrounding the passage to be occluded. Usually a pump cooperatively associated with a fluid reservoir is utilized to transfer fluid into and out of the cuff. As fluid is transferred into the cuff, the cuff inflates and closes the circumscribed body passage.

Artificial urinary sphincters (AUS) known in the state of the art consist of three major parts, namely the fluid reservoir, the cuff and a pump which is usually designated as the control mechanism of the AUS. The pump can be placed in a man's scrotum. It can also be placed underneath the skin in a woman's lower belly, labia or leg. Two conduit tubes connect all three major parts to each other. Use of an extra element, in particular of a conduit tube, increases the implantation time, complexity of the surgery and most importantly, the infection risk of a patient after implantation within the body.

A known problem with the inflatable artificial sphincters existing in the state of the art is the failure of the cuff in effectively occluding the excretory body passage when a sudden pressure increase occurs in the patient's abdomen. A sudden pressure increase may occur when, for instance, the patient laughs, coughs or is burst into laughter and also by way of certain physical movements such as bending the upper body down or when lifting a weight. In such cases, the normal pressure formed in the inflatable cuff may fail to effectively occlude the excretory body passage and excreted fluid which already accumulated behind the cuff or in the bladder may unintentionally leak outside the patient's body.

WO 2018/156092 A1 discloses an artificial sphincter comprising an inflatable occlusion means for occluding a body passage, a stretchable fluid reservoir and a pump means. The pump means has a first port in fluid communication with the occlusion means and a second port in fluid communication with the fluid reservoir for selectively transferring an isotonic fluid from the occlusion means to said reservoir to deflate said occlusion means. The artificial sphincter according to this piece of prior art needs to be improved further. The isotonic fluid loaded in the artificial sphincter may become contaminated in either during the surgical implantation or later on in time during use of the same. The contamination usually results in formation of solid particle content in the isotonic liquid. The solid particle content of contamination may clog the multiplicity of components in either of the pump or the inflatable balloons. These components of concern are usually check valves, flow retarders etc. Clogging in anyone of these components results in that the artificial sphincter becomes unfunctional and shall surgically be removed from the patient. The overall system needs to be simplified in order to avoid complexity and to increase reliable service life of the artificial sphincter.

### OBJECTS OF THE PRESENT INVENTION

Primary object of the present invention is to provide a new artificial sphincter which eliminates the drawbacks of the existing artificial sphincters.

In particular, an object of the present invention is to provide a new control mechanism for an artificial sphincter which effectively occludes the excretory body passage of a patient even when a sudden pressure increase occurs in the abdomen of the patient.

A further object of the present invention is to provide a new artificial sphincter which is simple and easy to manufacture.

A final object of the present invention is to provide a simplified artificial sphincter in which the pump is simplified, easy to manufacture and has a longer service life.

### BRIEF DESCRIPTIONS OF THE DRAWINGS

The figures whose brief explanations are herewith provided are solely intended for providing a better understanding of the present invention and are as such not intended to define the scope of protection or the context in which said scope is interpreted in the absence of the description.
Fig. 1 shows a perspective view of an artificial sphincter according to the present invention,
Fig. 2 shows the cross-sectional view of the artificial sphincter of Fig. 1 in the inflated state of the occlusion means,
Fig. 3 shows the cross-sectional view of the artificial sphincter of Fig. 1 in the deflated state of the occlusion means,
Fig. 4 shows the cross-sectional view of the semipermeable check valve given in Fig. 2,
Fig. 5A shows 2-dimensional cross-sectional view of the pressure sensitive check valve given in Fig. 2,
Fig. 5B shows 3D schematic view of the pressure sensitive check valve given in Fig. 2,
Fig. 6 shows a multiplicity of the perspective views of the cratered ball and its annular seating,
Fig. 7 shows a multiplicity of the perspective views of the ball and its recesses seating,
Fig. 8a shows frontal view of the pump means according to the present invention,
Fig. 8b shows side view of the pump means according to the present invention,
Fig. 8c shows the A-A cross-sectional view of Fig. 8b,
Fig. 9a shows the perspective of the cross-sectional view of a first embodiment of the pump means without a spring
Fig. 9b shows 2D cross-sectional view of the pump means of Fig. 9a in a state where the bulb is squeezed,
Fig. 10 shows the 2D cross-sectional view of a second embodiment of the pump means having a spring and, in a state, where the bulb is squeezed. Broken arrows show the flow direction of the pressurizing fluid unless the arrow is marked with the letter "x".
Fig. 11 shows the pump of Fig. 10 in a state where the bulb is released.
Fig. 12 shows the pump of Fig. 10 in the equilibrium state.

### DETAILED DESCRIPTION OF THE INVENTION

The list of reference numerals used in the appended drawings is as follows;
- 1: artificial sphincter
- 3: pressure sensitive check valve
- 6: pump bulb
- 7: pump
- 8: second tube
- 9: first tube
- 11: balloon holder
- 13: first port
- 14: second port
- 20: occlusion means
- 23: stretchable fluid reservoir
- 24: pressure compensation balloon
- 28: lumen
- 30: semipermeable check valve
- 32: pervious seat
- 33: ball
- 34: nest
- 35: ball
- 36: annular seating
- 37: crater
- 39: recessed seating

- 40: recess
- 41: gap
- 42: ball
- 43: nest
- 44: spring
- 45: cratered ball
- 60: check valve
- 61: spring
- 62: core
- 63: blockage seat
- 65: resilient lips
- 66: chamber
- 67: lumen
- 68: ball
- 70: ball seating
- 71: proximal end
- 72: distal end

Objects of the present invention are achieved by the features of Claim 1 in which an artificial sphincter (1) which, in use, contains a pressurizing fluid is disclosed.

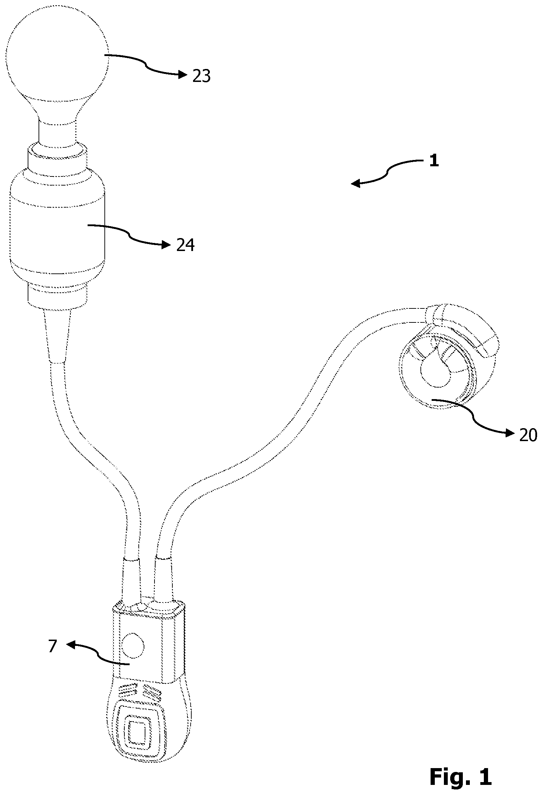

Fig. 1 shows the perspective view of an artificial sphincter (1) according to the present invention. The artificial sphincter (1) has a pump (7) located in between the occlusion means (20) and the stretchable fluid reservoir (23) for selectively transferring the pressurizing fluid. The pump (7) has a compressible bulb (6) for activation by the patient implanted with the artificial sphincter (1). The stretchable fluid reservoir (23) is in fluid communication with a pressure compensation balloon (24).

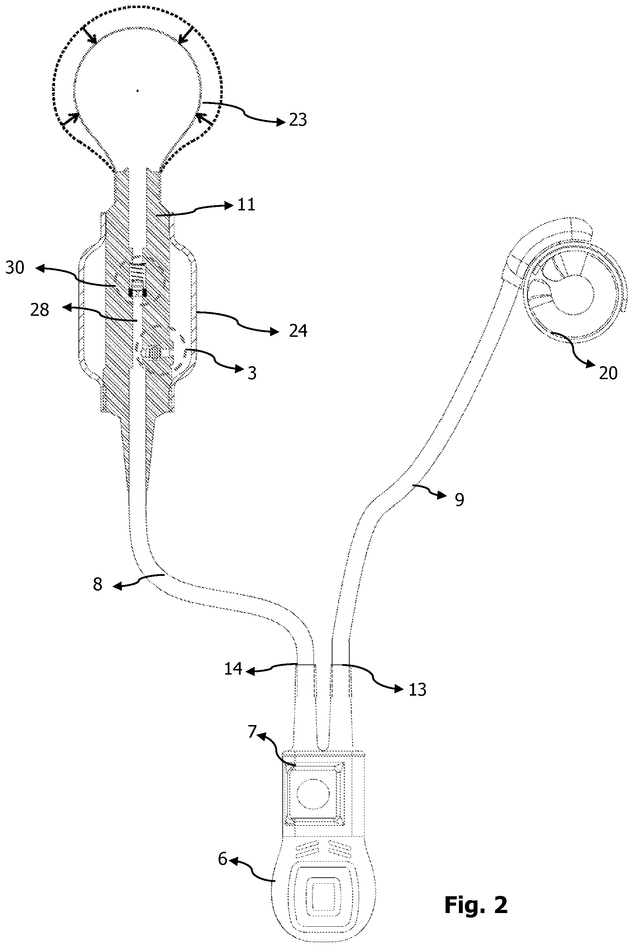

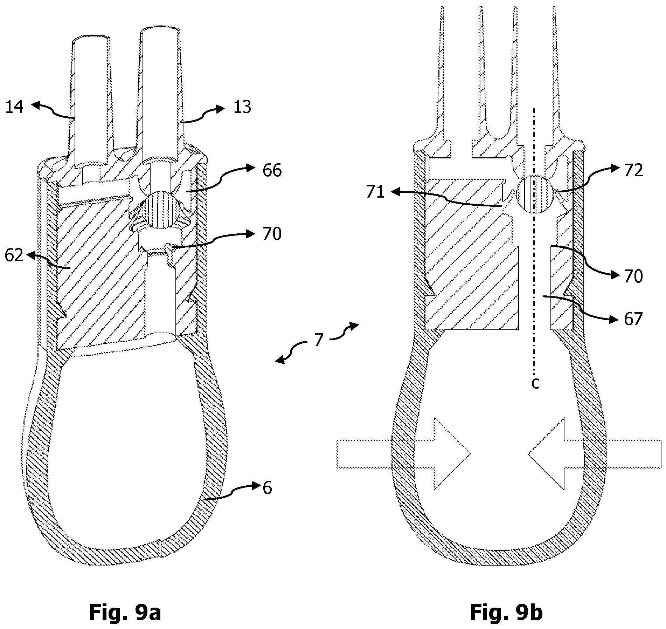

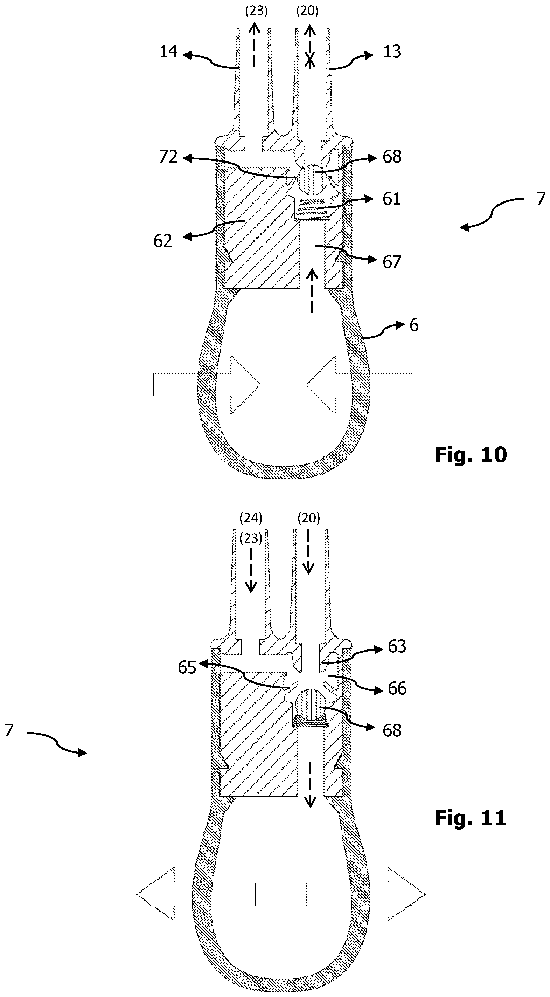

Fig. 2 shows the cross-sectional view of the artificial sphincter of Fig. 1 in the inflated state of the occlusion means (20). The occlusion means (20) is automatically and gradually inflated after it is deflated for allowing urination of the patient. When the bulb is squeezed or otherwise compressed, the pressurizing fluid contained in the bulb is transferred to the stretchable fluid reservoir (23). Once the bulb is squeezed, the ball (68) blocks the pathway towards the occlusion means (20). There is always free flow of the pressurizing fluid in between the pressure compensation balloon (24) and the occlusion means (20). Once the bulb (6) of the pump (7) is squeezed, as shown in Fig. 9b and Fig. 10, the pressurizing fluid contained in the bulb (6) is transferred to the stretchable fluid reservoir (23). Once the patient releases the bulb (6) as shown in Fig. 11, the pressurizing fluid flows from the pressure compensation balloon (24) towards the occlusion means (20) and towards the bulb (6). After a plurality of squeezing and releasing, all the fluid contained in the occlusion means (20) is transferred to the stretchable fluid reservoir (23).

In Fig. 2, the arrows shown around the stretchable fluid reservoir (23) implies that the stretchable fluid reservoir (23) shrinks due to transfer of the pressurizing fluid from the stretchable fluid reservoir (23) towards the pressure compensation balloon (24) and hence, towards the occlusion means (20). This transfer of fluid results from the abdominal pressure of the patient. The broken lines around the stretchable fluid reservoir (23) corresponds to state of the stretchable fluid reservoir (23) in the deflated state of the occlusions means (20).

The artificial sphincter (1) according to the present invention comprises an inflatable occlusion means (20) for occluding a body passage, a stretchable fluid reservoir (23) and a pump means (7) having a first port (13) in fluid communication with said occlusion means (20) via a first tube (9) and a second port (14) in fluid communication with said fluid reservoir (23) via a second tube (8) for selectively transferring the pressurizing fluid from said occlusion means to said reservoir to deflate said occlusion means so that a blocked body passage may be opened. The pump means has a compressible bulb (6). The artificial sphincter (1) further comprises a balloon holder (11) having a lumen (28) which establishes fluid communication in between said fluid reservoir (23) and said second tube (8). The artificial sphincter (1) has a pressure compensation balloon (24) which is in fluid communication with the lumen of said balloon holder (11) and which, in use, is to be implanted in the abdomen of a patient. The pump means (7) according to the present invention comprises a lumen (67), whose first end has a ball seating (70) and a ball (68) accommodated on said seating and whose second end is open to the bulb (6). The pump means (7) has a blockage seat (63) which is located in the entry of first port (13) and which is sized and shaped to block fluid flow towards the first port (13) when, the ball is forced to lean on the blockage seat (63) by the pressurized fluid coming from the bulb (6). The pump further has a chamber (66) in which the ball (68) is movable, said chamber being located in between blockage seat (63) and the ball seating (70). Characterizingly, the pump means (7) has one or more resilient lips (65) located circumferentially above (+y) the ball (68) in the chamber (66), wherein the annular cavity formed by the distal ends (72) of the resilient lips (65) has a diameter which is less than the diameter of the ball (68) so that the ball can be guided towards the blockage seat (63) by the resilient lips (65) when the bulb (6) is squeezed.

The one or more resilient lips (65) located circumferentially above (+y) the ball (68) in the chamber (66) has a proximal end (71) and a distal end (72) as shown in Fig. 9a and Fig. 9b. When the bulb (6) of the pump means (7) is squeezed as shown in Fig. 9b and Fig. 10, the pressurized fluid coming from the bulb (6) enters in the lumen (67) and pushes the ball (68) towards the blockage seat (63) for blocking the flow towards the occlusion means (20). Arrows shown in dashed lines imply the flow direction of the pressurizing fluid. If the arrow is marked with a letter "x", this implies that there exists no flow in the direction shown by the arrow.

Squeezing the bulb results that the pressurizing fluid contained in the bulb (6) passes around the ball (68) and enter the chamber (66) and then flow though the second port (14) towards the stretchable fluid reservoir (23). When the bulb (6) is squeezed or otherwise compressed, the resilient lips (65) bend further and allow the pressurizing fluid pass from around the ball (68) and enter the chamber (66) of the pump means (7).

While the ball (68) moves in the chamber (66) under the pressure of the pressurizing fluid, the ball (68) is guided by the distal ends (72) of the one or more resilient lips (65) towards the blockage seat (63). This guidance aims to perfectly block the entry of the first port (13) towards the occlusion means (20).

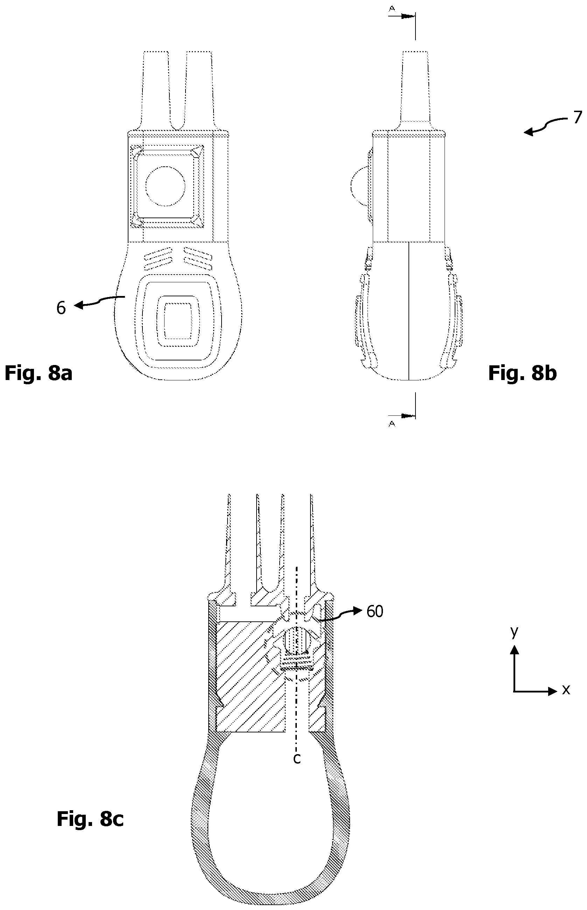

Distal ends (72) of the one more resilient lips (65) define an annular cavity. This cavity is in the form of a 2D circular plane. As shown in Fig. 8c and in Fig. 9b, the primary axis (c) of the annular cavity formed by the distal ends (72) of said one or more the resilient lips (65) and the primary axis (c) of the blockage seat (63) coincide with each other so that the ball (68) can be guided by the resilient lips (65) for perfectly blocking the blockage seat (63).

The annular cavity formed by the distal ends (72) of the resilient lips (65) has a diameter which is less than the diameter of the ball (68) so that the ball can be guided towards the blockage seat (63) by the resilient lips (65) when the bulb (6) is squeezed. Another important function of the resilient lips (65) is the elimination of the undesired blockage of the entry of the first port (13) during inflation of the occlusion means (20). Once the patient urinates, the artificial sphincter (1) automatically starts the inflation cycle of the occlusion means (20) and the pressurizing fluid flows from stretchable fluid reservoir (23) towards the occlusion means (20). During inflation, the pressure of the inflow towards the occlusion means (20) is slightly less than the pressure of the fluid found in the bulb (6). This slight pressure difference results that the ball (68) tends to move upwards (+y) in the chamber (66) and unwantedly block the blockage seat (63). If the entry of the first port (13) is unwantedly blocked, the inflation cycle cannot be completed. The rigidity of the resilient lips (65) is sufficient to prevent the ball (68) from unwantedly blocking the entry of the first port (13) arising from the slight pressure difference. However, the rigidity of the resilient lips (65) is not sufficient to prevent the ball (68) block the entry of the first port (13) when the bulb (6) squeezed by the patient.

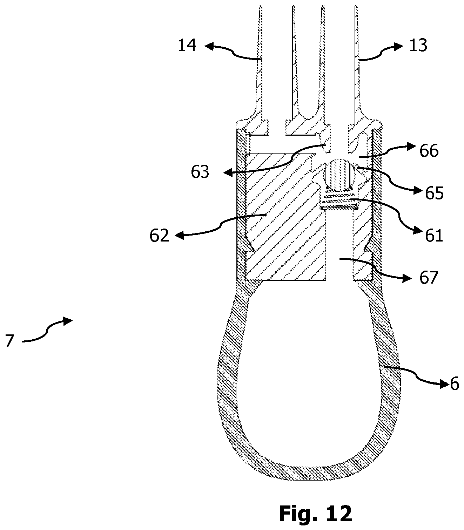

The check valve (60) of the pump means (7) has a first position in which the ball (68) leans on the blockage seat (63) and blocks the flow towards the occlusion means (20) when the bulb (6) is squeezed. The check valve (60) has a second position in which the ball (68) sits and compresses the spring (61) and allow fluid flow towards the bulb (6) when the bulb is released as shown in Fig. 11. The check valve (60) has a third position in which the ball (68) floats freely underneath said one or more resilient lips (65) when the occlusion means (20) is inflated and the system approaches equilibrium state as shown in Fig. 12.

Fig. 8a shows frontal view of the pump means according to the present invention whereas Fig. 8b shows side view. Fig. 8c shows the A-A cross-sectional view of Fig. 8b. In this embodiment, the check valve of the pump has a spring. The spring (61) accommodated on the ball seating (70) supports the ball (68) towards the one or more resilient lips (65). Use of a spring (61) in the check vale (60) of the pump means (7) is optional and the sphincter (1) and in particular the pump means (7) functions perfectly well in the absence of the spring (61). The first embodiment which does not have the spring is depicted in Fig. 9a and Fig. 9b.

Fig. 11 shows the flow pathway of the pressurizing fluid in the artificial sphincter (1) during deflation of the occlusion means (20). The pressurizing fluid contained in the occlusion means (20) flows, via the first tube (9), in the bulb (6) of the pump (7). During deflation, due to the abdominal pressure, the pressurizing fluid contained in the pressure compensation balloon (24) and contained in the stretchable fluid reservoir (23) may also flow towards the bulb (6) of the pump (7). However, each time the bulb is compressed as shown in Fig. 9b and Fig. 10, the fluid in the bulb flows towards the stretchable fluid reservoir (23). As the flow towards the occlusion means (20) is blocked by the check valve (60) as depicted in Fig. 10, the act of squeezing and releasing the bulb results in evacuation of the fluid contained in the occlusion means (20). In this state, the occlusion means (20) unblock the body canal and the patient may freely urinate or defecate.

During automatic inflation of the occlusion means (20), the pressurizing fluid found in the stretchable fluid reservoir (23) can no longer flow into the pump bulb when the bulb (6) is full. After the bulb (6) is full of the pressurizing fluid, the pressurizing fluid contained in the stretchable fluid reservoir (23) will first gradually flow towards the occlusion means (20). Once the occlusion means (20) is fully inflated, the pressurizing fluid found in the stretchable fluid reservoir (23) will finally flow into the pressure compensation balloon (24) and inflate the same. In this position, the occlusion means (20) is fully inflated and the patient is continent again. As the artificial sphincter (1) approaches the equilibrium state, the fluid pressure in the stretchable fluid reservoir (23) equals to the pressure in the compensation balloon (24) and in the occlusion means (20).

A balloon holder (11) establishes fluid communication in between the stretchable fluid reservoir (23) and the pressure compensation balloon (24). The balloon holder (11) has a lumen (28) at one end of which the stretchable fluid reservoir (23) is attached. The other end of the lumen (28) of the balloon holder (11) is connected to pump (7) via a second tube (8).

The stretchable fluid reservoir (23) is made of a resilient material which may expand by way of stretching such that, in the stretched state, the pressurizing fluid contained in said reservoir (23) has a pressure more than the abdominal pressure of the patient due to stretching of the resilient material. The automatic and gradual transfer of the pressurizing fluid from the stretchable fluid reservoir (23) towards the occlusions means is activated by the internal pressure of the stretchable fluid reservoir (23).

A semipermeable check valve (30) is located in the lumen (28) of the balloon holder (11). The semipermeable check valve (30) fully allows fluid flow coming from the second tube (8) pass towards the stretchable fluid reservoir (23). However, the semipermeable check valve (30) allows only a limited flow rate in the reverse direction, i.e. fluid flow from the stretchable fluid reservoir (23) towards the second tube (8) or the rest of the lumen (28) of the balloon holder (11). When the pressurizing fluid comes from the second tube (8), for example during deflation of the occlusion means (20), the fluid pushes the ball (35) towards the stretchable fluid reservoir (23) resulting in compression of the spring (44). Once the spring is compressed, all fluid coming from the second tube (8) pass around the ball (35) and flow into the stretchable fluid reservoir (23).

A pressure sensitive check valve (3) is located in the lumen (28) of the balloon holder (11). The pressure sensitive check valve (3) establishes fluid communication in between the lumen (11) and the pressure compensation balloon (24). The pressure sensitive check valve (3) allows all fluid flow coming from the pressure compensation balloon (24) towards the lumen (28) and hence towards the occlusion means (20) via the second tube (8) and the first tube (9).

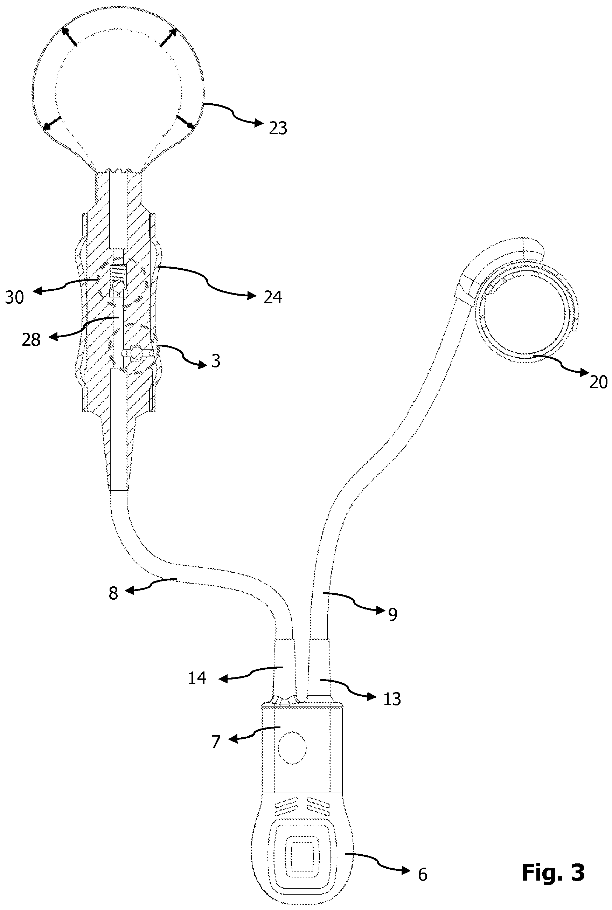

Fig. 3 shows the cross-sectional view of the artificial sphincter of Fig. 1 in the deflated state of the occlusion means (20). The occlusion means (20) is connected to first port (13) of the pump (7) via the first tube (9). The pressurizing fluid contained in the occlusion means (20) during its inflated state is transferred, via the first tube (9), partially in the bulb (6) of the pump (7) and partially in the stretchable fluid reservoir (23). The arrows shown around the stretchable fluid reservoir (23) imply that the volume of the stretchable fluid reservoir (23) has increased due to the additional volume of the pressurizing fluid transferred from the occlusion means (20), from the bulb (6) of the pump (7).

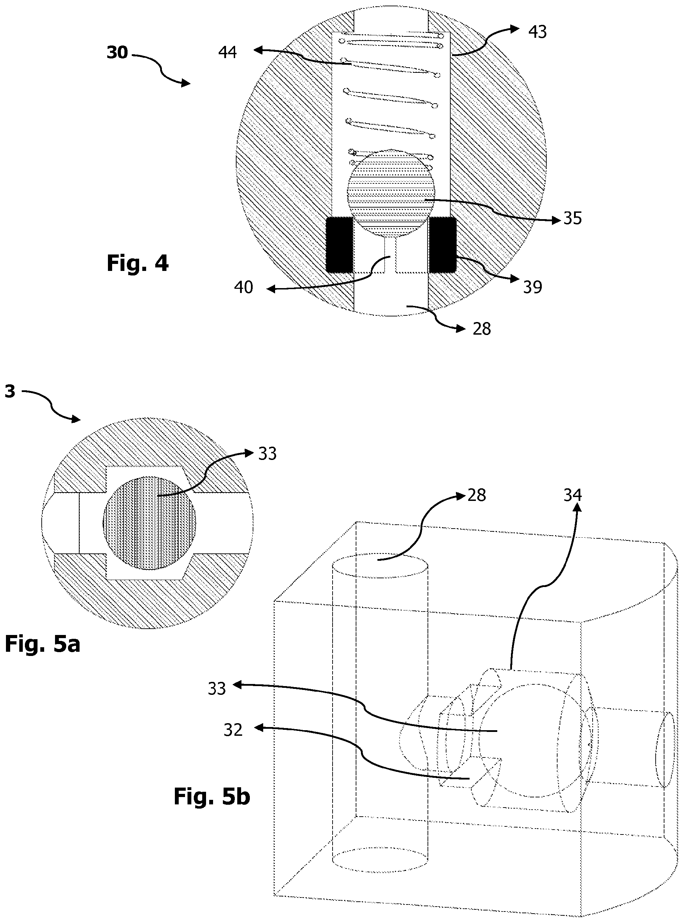

Fig. 4 shows the cross-sectional view of the semipermeable check valve (30) depicted in Fig. 2 and in Fig. 3. The semipermeable check valve (30) may comprise a spring (44) and a ball (35) which is accommodated on a recessed seating (39). The recessed seating (39) has a recess (40) for allowing restricted fluid flow from the stretchable fluid reservoir (23) towards the lumen (28) of the balloon holder (11). While the semipermeable check valve (30) allows all fluid flow coming from the lumen (28) towards the stretchable fluid reservoir (23), the recess (40) allows only very restricted flow rate in the reverse direction.

While the semipermeable check valve (30) given in Fig. 4 comprises a conventional combination of a spring (44) and a ball (35) accommodated on a seating, it may be composed of other check valve configurations. What makes the semipermeable check valve (30) unique and in particular, "semipermeable" is the recess (40) which allows limited fluid flow in the reverse direction, i.e. from the stretchable fluid reservoir (23) towards the lumen (28) of the balloon holder (11).

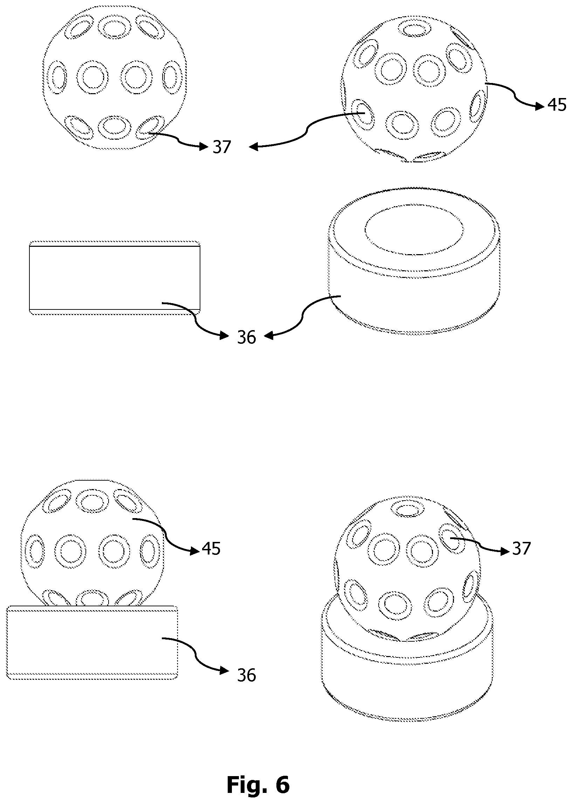

An alternative embodiment of the semipermeable check valve (30) is shown in Fig. 6 in which a cratered ball (45) is accommodated on an annular seating (36). The cratered ball (45) has several craters (37) on its outer surface such that the restricted reverse flow of the fluid from the stretchable fluid reservoir (23) can be established through the gaps in between the craters and the annular seating (36). Despite the fact that there is no need to establish a recess (40) in the annular seating (36), one or more recesses (40) may be formed on the annular seating (36) if the flow rate of the reverse flow needs to be increased.

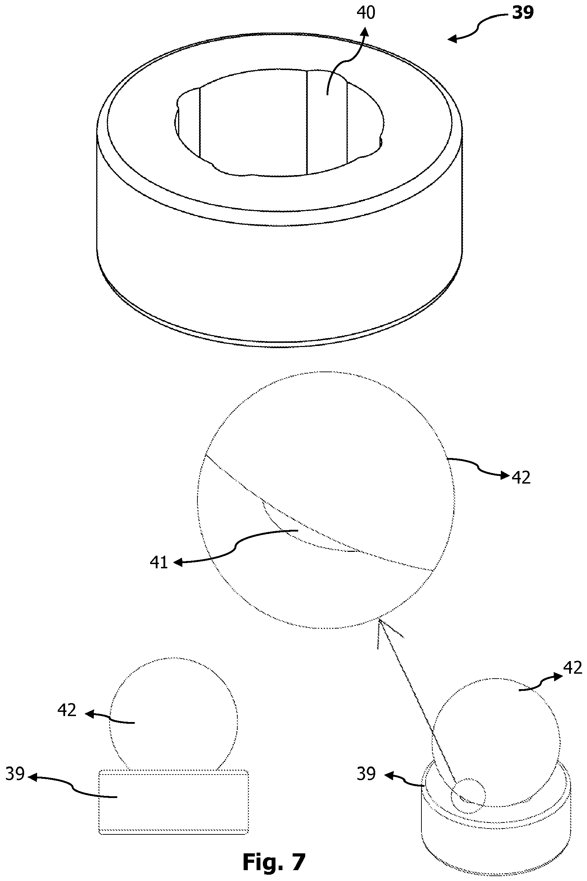

Still a further alternative embodiment of the semipermeable check valve (30) is shown in Fig. 7 in which a ball having smooth surface is accommodated on the recessed seating (39) having multiple recesses (40). In this configuration, the reverse flow, i.e. the flow from the stretchable fluid reservoir (23) towards the lumen (28) can be established through multiple gaps in between the recesses (40) of the recessed seating (39) and the ball (42).

Fig. 5a shows 2D cross sectional view of the pressure sensitive check valve given in Fig. 2 and in Fig. 3 while Fig. 5b shows 3D schematic perspective view of the same pressure sensitive check valve (3). The pressure sensitive check valve (3) is located in between the pressure compensation balloon (24) and the lumen of said balloon holder (11). The pressure sensitive check valve (3) always allows fluid flow from the pressure compensation balloon (24) towards the lumen (28) so that the fluid contained in the pressure compensation balloon (24) is freely flowable towards the inflatable occlusion means (20) and, in use, a sudden pressure increase which occurs in the abdomen of a patient is instantly conveyed to the inflatable occlusion means (20) for prevention of leaks.

The pressure sensitive check valve (3) comprises a ball (33) which floats freely in a nest (34). The density of the ball (33) is substantially close to the density of the pressurizing fluid contained in the artificial sphincter (1) proposed by the present invention. The nest (34) of the pressure sensitive check valve (3) has a pervious seat (32), as shown in Fig. 5b, at its end which is closer to the lumen (28) of the balloon holder (11). The geometry of the pervious seat (32) ensures that fluid flow towards the lumen is always guaranteed even if the ball (33) leans on the pervious seat (32) which has rectangular barriers, as shown in Fig. 5b, for ensuring passage of the fluid around the ball (33). The nest (34) has a smooth circular seat which may be blocked by the ball (33) at its other end when the fluid pressure in the lumen (28) is greater than the fluid pressure in the pressure compensation balloon (24). The check valve (3) is designated as "a pressure sensitive check valve (3)" because it blocks fluid flow towards the pressure compensation balloon (24) only when the fluid pressure in pressure compensation balloon (24) is substantially less than the fluid pressure in the lumen (28) of the balloon holder (11).

The pressure sensitive check valve (3) located in the lumen (28) of the balloon holder (11) is proximal to the second tube (8) as compared to the semipermeable check valve (30).

Reverting back to Fig. 2 in which the occlusion means (20) is depicted in the inflated state, the pressure compensation balloon (24) is in its inflated form and the stretchable fluid reservoir (23) is in its shrinked form. In this state, i.e. the occlusion means in its inflated state, the pressurizing fluid can freely flow towards the occlusion means (20) at any time. The pressure sensitive check valve (3) always allows fluid flow from the pressure compensation balloon (24) towards the lumen (28) and hence towards the occlusion means (20). Since the fluid contained in the pressure compensation balloon (24) is freely flowable towards the inflatable occlusion means (20), a sudden pressure increase which occurs in the abdomen of the patient is instantly conveyed to the inflatable occlusion means (20) resulting in better leak prevention.

Fig. 3 shows the cross-sectional view of the artificial sphincter of Fig. 1 in the deflated state of the occlusion means (20). While the pressure compensation balloon (24) in Fig. 3 is in shrinked form, inflation of the pressure compensation balloon (24) will start simultaneously with the inflation of the occlusion means (20). When the occlusion means (20) is inflated, the system reaches an equilibrium state in which the fluid pressure in the occlusion means (20), in the stretchable fluid reservoir (23) and in the pressure compensation balloon (24) are stabilized and are equal to each other.

The equilibrium state corresponds to a state where the body canal is occluded and the patient has established his or her continence. Reverting back Fig. 5a and 5b, the ball (33) floats freely in the nest (34) once the equilibrium state is almost reached. This means that the pressure sensitive check valve (3) no longer blocks the flow of the pressurizing fluid in both directions. Once the equilibrium state is approached, the pressure compensation balloon (24) inflates and takes the inflated form as shown in Fig. 2. The pump means (7) in the equilibrium state is depicted in Fig. 12.

The equilibrium state is established by the semipermeable check valve (30) which allows restricted flow from the stretchable fluid reservoir (23) towards the lumen (28) of the balloon holder (11). The over pressure in the stretchable fluid reservoir (23) is smoothly and slowly reduced by the semipermeable check valve (30) which establishes the restricted reverse flow out of the stretchable fluid reservoir (23). Once the fluid pressure in the lumen (28) smoothly and slowly stabilizes, the pressure sensitive check valve (3) unblocks the flow towards the pressure compensation balloon (24) and inflates the same. The fluid pressure in the occlusion means (20), in the stretchable fluid reservoir (23) and in the pressure compensation balloon (24) stabilizes and the equilibrium state is reached with a fully inflated occlusion means (20).

The occlusion means (20) of the artificial sphincter (1) according to the present invention is sized and shaped to occlude an anal or urethral canal of a human being.

### Deflation of the Occlusion Means:

When the patient needs to urinate, the patient shall squeeze and release the bulb (6) of the pump a few times, typically 3 or 4 times is sufficient to transfer all fluid contained in the occlusion means (20) towards the stretchable fluid reservoir (23). When the bulb is squeezed as shown in Fig. 9b and Fig. 10, the fluid inside the bulb goes to the stretchable fluid reservoir (23). Squeezing the bulb results that the pressurizing fluid contained in the bulb (6) passes around the ball (68) and enter the chamber (66) and then flow though the second port (14) towards the stretchable fluid reservoir (23). When the bulb (6) is squeezed or otherwise compressed, the resilient lips (65) bend further and allow the pressurizing fluid pass from around the ball (68).

There is no fluid flow towards the occlusion means (20) as the pump has a check valve (60) preventing flow towards the occlusion means (20). However, when the bulb (6) is released as shown in Fig. 11, the pressurizing fluid found in the occlusion means (20) is sucked into the bulb (6). When the bulb is released as shown in Fig. 11, the bulb (6) also sucks the fluid contained in the pressure compensation balloon (24). The check valve (60) allows the fluid coming from the pressure compensation balloon (24) fill in the bulb (6) of the pump (7). As each time the bulb is squeezed and released, the fluid contained in the occlusion means (20) decrease in volume and after typically 3-5 times of squeeze & release, the occlusion means (20) has no pressurizing fluid at all. The patient is now free to urinate.

### Inflation of the Occlusion Means:

The occlusion means is automatically inflated by the abdominal pressure of the patient as well as by the pressure increase arising from the stretching of the walls of the stretchable fluid reservoir (23). Since the fluid previously found in the occlusion means (20) were transferred into the stretchable fluid reservoir (23), the internal pressure of the stretchable fluid reservoir (23) is very high not only because the reservoir (23) is in its stretched form but also because of the abdominal pressure of the patient. Inflation of the occlusion means (20) occur gradually because of the fact that the semipermeable check valve (30) allows only a restricted reverse fluid flow towards the lumen (28) of the balloon holder (11). The semipermeable check valve (30) has tiny recesses (40) which retard the reverse flow from the stretchable fluid reservoir (23) towards the lumen (28). However, the restricted reverse fluid flow simultaneously fills the bulb (6) and the occlusion means (20). Once the bulb (6) is full, the reverse flow from the stretchable fluid reservoir (23) starts inflating the occlusion means (20). While the bulb (6) may be squeezed and shrinked in size, the bulb (6) cannot expand more than its initial volume.

Once the restricted flow from the stretchable fluid reservoir (23) fully inflates the occlusion means (20), the artificial sphincter (1) according to the present invention reaches is almost-stable form and finally the reverse flow from the stretchable fluid reservoir (23) flows into the pressure compensation balloon (24) and fills in the same as depicted in Fig. 2. The patient is now continent and the artificial sphincter (1) is now sensitive to sudden abdominal pressure changes which occur during laughing, coughing etc. The gradual and automatic inflation of the occlusion means (20) completes typically in around 3 minutes, during which the patient may freely urinate.

## Claims

1. An artificial sphincter (1) containing, in use, a pressurizing fluid, said artificial sphincter (1) comprising:
an inflatable occlusion means (20) for occluding a body passage,
a stretchable fluid reservoir (23),
a pump means (7) having a first port (13) in fluid communication with said occlusion means (20) via a first tube (9) and a second port (14) in fluid communication with said fluid reservoir (23) via a second tube (8) for selectively transferring the pressurizing fluid from said occlusion means to said reservoir to deflate said occlusion means so that a blocked body passage may be opened;
a compressible bulb (6) which is attached to the pump means (7) and which, in use, contains part of the pressurizing fluid,
a balloon holder (11) having a lumen (28) which establishes fluid communication in between said fluid reservoir (23) and said second tube (8),
a pressure compensation balloon (24) which is in fluid communication with the lumen of said balloon holder (11) and which, in use, is to be implanted in the abdomen of a patient,
wherein said pump means (7) comprises;
a lumen (67), whose first end has a ball seating (70) and a ball (68) accommodated on said seating and whose second end is open to the bulb (6),
a blockage seat (63) which is located in the entry of first port (13) and which is sized and shaped to block fluid flow towards the first port (13) when, the ball is forced to lean on the blockage seat (63) by the pressurized fluid coming from the bulb (6),
a chamber (66) in which the ball (68) is movable, said chamber being located in between blockage seat (63) and the ball seating (70),
wherein, when the bulb (6) is squeezed, the pressurizing fluid contained in the bulb (6) passes around the ball (68) and enters the chamber (66) and then flows through the second port (14) towards the stretchable fluid reservoir (23), **characterized by** one or more resilient lips (65) located circumferentially above (+y) the ball (68) in the chamber (66), wherein the annular cavity formed by the distal ends (72) of the resilient lips (65) has a diameter which is less than the diameter of the ball (68) so that the ball can be guided towards the blockage seat (63) by the resilient lips (65) when the bulb (6) is squeezed.

2. An artificial sphincter (1) according to Claim 1 wherein primary axis (c) of the annular cavity formed by the distal ends (72) of said one or more the resilient lips (65) and the primary axis (c) of the blockage seat (63) coincide with each other so that the ball (68) can be guided by the resilient annular lips (65) for tightly blocking the blockage seat (63).

3. An artificial sphincter (1) according to Claim 1 wherein the ball seating (70) has a spring (61) supporting the ball (68) towards the one or more resilient lips (65).

4. An artificial sphincter (1) according to Claim 1 wherein the artificial sphincter (1) further comprises a semipermeable check valve (30) which is located in the lumen (28) of said balloon holder (11), wherein said semipermeable check valve (30) always allows fluid flow coming from the second tube (8) to pass towards said stretchable fluid reservoir (23) and wherein said semipermeable check valve (30) only allows a restricted fluid flow from the stretchable fluid reservoir (23) towards the second tube (8),

5. An artificial sphincter (1) according to Claim 4 wherein the artificial sphincter (1) further comprises a pressure sensitive check valve (3) in between the pressure compensation balloon (24) and the lumen of said balloon holder (11), wherein said pressure sensitive check valve (3) always allows fluid flow from the pressure compensation balloon (24) towards the lumen (28) so that the fluid contained in the pressure compensation balloon is freely flowable towards the inflatable occlusion means (20) and, in use, a sudden pressure increase which occurs in the abdomen of a patient is instantly conveyed to the inflatable occlusion means (20).

6. An artificial sphincter (1) according to Claim 4 wherein the semipermeable check valve (30) has a recessed seating (39) having one or more recesses (40) so that a restricted fluid flow from the stretchable fluid reservoir (23) towards the second tube (8) is established through the recesses (40).

7. An artificial sphincter (1) according to Claim 4 wherein the semipermeable check valve (30) has an annular seating (16) and a cratered ball (45) having a plurality of craters (37) so that a restricted fluid flow from the stretchable fluid reservoir (23) towards the second tube (8) is established through the craters (37).

8. An artificial sphincter (1) according to Claim 5 wherein the pressure sensitive check valve (3) has a ball (33) located inside a nest (34), said ball allowing fluid flow from the lumen (28) towards the pressure compensation balloon (24) only during inflation of the occlusion means (20).

9. An artificial sphincter (1) according to Claim 5 wherein the pressure sensitive check valve (3) has a pervious seat (32) which is located in between the ball (33) and the lumen (28) and which ensures that the ball (33) always allows, due to its spherical geometry, passage of fluid flow from the pressure compensation balloon (24) towards the lumen (28).

10. An artificial sphincter (1) according to Claim 4 wherein the ball (33) has a density which is substantially equal to the density of the pressurizing fluid so that the ball freely floats freely in said nest (34).

11. An artificial sphincter (1) according to Claim 4 wherein the reservoir (23) is made of a resilient material which may expand by way of stretching such that, in the stretched state, the pressurizing fluid contained in said reservoir (23) has a pressure more than the abdominal pressure of the patient due to stretching of the resilient material.

12. An artificial sphincter (1) according to Claim 1 wherein the occlusion means (20) is sized and shaped to occlude an anal or urethral canal of a human being.

## Patentansprüche

1. Künstlicher Sphinkter (1), der im Einsatz ein unter Druck stehendes Fluid enthält, wobei der künstliche Sphinkter (1) umfasst:
ein aufblasbares Okklusionsmittel (20) zum Verschließen eines Körperdurchgangs,
ein dehnbares Fluidreservoir (23),
eine Pumpeneinrichtung (7) mit einer ersten Öffnung (13), die über einen ersten Schlauch (9) in Fluidverbindung mit dem Okklusionsmittel (20) steht, und einer zweiten Öffnung (14), die über einen zweiten Schlauch (8) in Fluidverbindung mit dem Fluidreservoir (23) steht, um das Druckfluid selektiv von dem Okklusionsmittel zum Reservoir zu übertragen, um das Okklusionsmittel zu entleeren, so daß ein blockierter Körperdurchgang geöffnet werden kann;
einen komprimierbaren Ball (6), der an der Pumpeneinrichtung (7) befestigt ist und der im Einsatz einen Teil des Druckfluids enthält,
einen Ballonhalter (11) mit einem Lumen (28), das eine Fluidverbindung zwischen dem Fluidreservoir (23) und dem zweiten Schlauch (8) herstellt,
einen Druckausgleichsballon (24), der in Fluidverbindung mit dem Lumen des Ballonhalters (11) steht und der bei der Verwendung in das Abdomen eines Patienten implantiert werden soll,
wobei die Pumpeneinrichtung (7) umfasst;
ein Lumen (67), dessen erstes Ende einen Kugelsitz (70) und eine auf diesem Sitz untergebrachte Kugel (68) aufweist und dessen zweites Ende zum Ball (6) hin offen ist,
einen Blockiersitz (63), der sich im Eingang der ersten Öffnung (13) befindet und der so bemessen und geformt ist, dass er den Flüssigkeitsstrom zur ersten Öffnung (13) blockiert, wenn die Kugel durch das aus dem Ball (6) kommende Druckfluid gezwungen wird, sich auf den Blockiersitz (63) zu stützen,
eine Kammer (66), in der die Kugel (68) beweglich ist, wobei die Kammer zwischen dem Blockiersitz (63) und dem Kugelsitz (70) angeordnet ist,
wobei, wenn der Ball (6) zusammengedrückt wird, das in dem Ball (6) enthaltene Druckfluid um die Kugel (68) herumfließt und in die Kammer (66) eintritt und dann durch die zweite Öffnung (14) in Richtung des dehnbaren Fluidreservoirs (23) fließt, **gekennzeichnet durch** eine oder mehrere elastische Lippen (65), die in Umfangsrichtung über (+y) der Kugel (68) in der Kammer (66) angeordnet sind, wobei der ringförmige Hohlraum, der durch die distalen Enden (72) der elastischen Lippen (65) gebildet wird, einen Durchmesser hat, der kleiner ist als der Durchmesser der Kugel (68), so dass die Kugel durch die elastischen Lippen (65) zum Blockiersitz (63) geführt werden kann, wenn der Ball (6) zusammengedrückt wird.

2. Künstlicher Sphinkter (1) nach Anspruch 1, wobei die Primärachse (c) des ringförmigen Hohlraums, der durch die distalen Enden (72) der einen oder mehreren elastischen Lippen (65) gebildet wird, und die Primärachse (c) des Blockiersitzes (63) miteinander übereinstimmen, so dass die Kugel (68) von den elastischen ringförmigen Lippen (65) geführt werden kann, um den Blockiersitz (63) fest zu blockieren.

3. Künstlicher Sphinkter (1) nach Anspruch 1, wobei der Kugelsitz (70) eine Feder (61) aufweist, die die Kugel (68) in Richtung der einen oder mehreren elastischen Lippen (65) stützt.

4. Künstlicher Sphinkter (1) nach Anspruch 1, wobei der künstliche Sphinkter (1) ferner ein halbdurchlässiges Rückschlagventil (30) umfasst, das sich im Lumen (28) des Ballonhalters (11) befindet, wobei das halbdurchlässige Rückschlagventil (30) stets einen vom zweiten Schlauch (8) kommenden Flüssigkeitsstrom in Richtung des dehnbaren Fluidreservoirs (23) durchlässt und wobei das halbdurchlässige Rückschlagventil (30) nur einen begrenzten Flüssigkeitsstrom aus dem dehnbaren Fluidreservoir (23) in Richtung des zweiten Schlauchs (8) zulässt,

5. Künstlicher Sphinkter (1) nach Anspruch 4, wobei der künstliche Sphinkter (1) ferner ein druckempfindliches Rückschlagventil (3) zwischen dem Druckausgleichsballon (24) und dem Lumen des Ballonhalters (11) aufweist, wobei das druckempfindliche Rückschlagventil (3) stets einen Flüssigkeitsstrom von dem Druckausgleichsballon (24) zu dem Lumen (28) zulässt, so dass die in dem Druckausgleichsballon enthaltene Flüssigkeit frei zu dem aufblasbaren Okklusionsmittel (20) fließen kann und im Einsatz ein plötzlicher Druckanstieg, der im Bauchraum eines Patienten auftritt, sofort zu dem aufblasbaren Okklusionsmittel (20) geleitet wird.

6. Künstlicher Sphinkter (1) nach Anspruch 4, wobei das semipermeable Rückschlagventil (30) einen ausgesparten Sitz (39) mit einer oder mehreren Aussparungen (40) aufweist, so dass durch die Aussparungen (40) ein begrenzter Flüssigkeitsstrom aus dem dehnbaren Fluidreservoir (23) in Richtung des zweiten Schlauchs (8) hergestellt wird.

7. Künstlicher Sphinkter (1) nach Anspruch 4, wobei das halbdurchlässige Rückschlagventil (30) einen ringförmigen Sitz (16) und eine mit Kratern versehene Kugel (45) mit einer Vielzahl von Kratern (37) aufweist, so dass ein begrenzter Flüssigkeitsstrom von dem dehnbaren Fluidreservoir (23) zu dem zweiten Schlauch (8) durch die Krater (37) hergestellt wird.

8. Künstlicher Sphinkter (1) nach Anspruch 5, wobei das druckempfindliche Rückschlagventil (3) eine Kugel (33) aufweist, die sich in einem Nest (34) befindet, wobei die Kugel nur während des Aufblasens der Okklusionsmittel (20) einen Flüssigkeitsstrom vom Lumen (28) zum Druckausgleichsballon (24) ermöglicht.

9. Künstlicher Sphinkter (1) nach Anspruch 5, wobei das druckempfindliche Rückschlagventil (3) einen durchlässigen Sitz (32) aufweist, der sich zwischen der Kugel (33) und dem Lumen (28) befindet und der sicherstellt, dass die Kugel (33) aufgrund ihrer kugelförmigen Geometrie stets einen Flüssigkeitsstrom vom Druckausgleichsballon (24) zum Lumen (28) durchlässt.

10. Künstlicher Sphinkter (1) nach Anspruch 4, wobei die Kugel (33) eine Dichte aufweist, die im wesentlichen gleich der Dichte des Druckfluids ist, so daß die Kugel frei in dem Nest (34) schwimmt.

11. Künstlicher Sphinkter (1) nach Anspruch 4, wobei das Reservoir (23) aus einem elastischen Material besteht, das sich durch Dehnung ausdehnen kann, so dass das in dem Reservoir (23) enthaltene Druckfluid im gedehnten Zustand aufgrund der Dehnung des elastischen Materials einen höheren Druck als der abdominale Druck des Patienten aufweist.

12. Künstlicher Sphinkter (1) nach Anspruch 1, wobei das Okklusionsmittel (20) so bemessen und geformt ist, dass es einen Analkanal oder eine Harnröhre eines Menschen verschließt.

## Revendications

1. - Sphincter artificiel (1) contenant, en utilisation, un fluide de pressurisation, ledit sphincter artificiel (1) comprenant :
un moyen d'occlusion gonflable (20) pour occlure un passage corporel,
un réservoir de fluide extensible (23),
un moyen de pompage (7) ayant un premier orifice (13) en communication fluidique avec ledit moyen d'occlusion (20) via un premier tube (9) et un second orifice (14) en communication fluidique avec ledit réservoir de fluide (23) via un second tube (8) pour transférer de manière sélective le fluide de pressurisation dudit moyen d'occlusion audit réservoir pour dégonfler ledit moyen d'occlusion de telle sorte qu'un passage corporel bloqué peut être ouvert ;
une poire compressible (6) qui est attachée au moyen de pompage (7) et qui, en utilisation, contient une partie du fluide de pressurisation,
un porte-ballonnet (11) ayant une lumière (28) qui établit une communication fluidique entre ledit réservoir de fluide (23) et ledit second tube (8),
un ballonnet de compensation de pression (24) qui est en communication fluidique avec la lumière dudit porte-ballonnet (11) et qui, en utilisation, est destiné à être implanté dans l'abdomen d'un patient,
ledit moyen de pompage (7) comprenant :
une lumière (67), dont la première extrémité comporte un siège de bille (70) et une bille (68) reçue sur ledit siège, et dont la seconde extrémité débouche sur la poire (6),
un siège de blocage (63) qui est situé dans l'entrée du premier orifice (13) et qui est dimensionné et formé pour bloquer un écoulement de fluide vers le premier orifice (13) lorsque la bille est contrainte de s'appuyer sur le siège de blocage (63) par le fluide pressurisé provenant de la poire (6),
une chambre (66) dans laquelle la bille (68) est mobile, ladite chambre étant située entre le siège de blocage (63) et le siège de bille (70),
lorsque la poire (6) est pressée, le fluide de pressurisation contenu dans la poire (6) passe autour de la bille (68) et entre dans la chambre (66), puis s'écoule par le second orifice (14) vers le réservoir de fluide extensible (23), **caractérisé par** une ou plusieurs lèvres élastiques (65) situées de manière circonférentielle au-dessus (+y) de la bille (68) dans la chambre (66), la cavité annulaire formée par les extrémités distales (72) des lèvres élastiques (65) ayant un diamètre qui est inférieur au diamètre de la bille (68), de telle sorte que la bille peut être guidée vers le siège de blocage (63) par les lèvres élastiques (65) lorsque la poire (6) est pressée.

2. - Sphincter artificiel (1) selon la revendication 1, dans lequel un axe primaire (c) de la cavité annulaire formée par les extrémités distales (72) de ladite ou desdites lèvres élastiques (65) et l'axe primaire (c) du siège de blocage (63) coïncident l'un avec l'autre de telle sorte que la bille (68) peut être guidée par les lèvres annulaires élastiques (65) pour bloquer hermétiquement le siège de blocage (63).

3. - Sphincter artificiel (1) selon la revendication 1, dans lequel le siège de bille (70) comporte un ressort (61) supportant la bille (68) vers la ou les lèvres élastiques (65).

4. - Sphincter artificiel (1) selon la revendication 1, dans lequel le sphincter artificiel (1) comprend en outre un clapet de non-retour semi-perméable (30) qui est situé dans la lumière (28) dudit porte-ballonnet (11), ledit clapet de non-retour semi-perméable (30) permettant toujours à un écoulement de fluide provenant du second tube (8) de passer vers ledit réservoir de fluide extensible (23), et ledit clapet de non-retour semi-perméable (30) permettant uniquement un écoulement de fluide restreint du réservoir de fluide extensible (23) vers le second tube (8).

5. - Sphincter artificiel (1) selon la revendication 4, le sphincter artificiel (1) comprenant en outre un clapet de non-retour sensible à la pression (3) entre le ballonnet de compensation de pression (24) et la lumière dudit porte-ballonnet (11), ledit clapet de non-retour sensible à la pression (3) permettant toujours un écoulement de fluide du ballonnet de compensation de pression (24) vers la lumière (28) de telle sorte que le fluide contenu dans le ballonnet de compensation de pression peut s'écouler librement vers le moyen d'occlusion gonflable (20) et, en utilisation, une augmentation soudaine de pression qui se produit dans l'abdomen d'un patient est instantanément transmise jusqu'au moyen d'occlusion gonflable (20).

6. - Sphincter artificiel (1) selon la revendication 4, dans lequel le clapet de non-retour semi-perméable (30) comporte un siège évidé (39) ayant un ou plusieurs évidements (40) de telle sorte qu'un écoulement de fluide restreint du réservoir de fluide extensible (23) vers le second tube (8) est établi par l'intermédiaire des évidements (40).

7. - Sphincter artificiel (1) selon la revendication 4, dans lequel le clapet de non-retour semi-perméable (30) comporte un siège annulaire (16) et une bille couverte de cratères (45) ayant une pluralité de cratères (37) de telle sorte qu'un écoulement de fluide restreint du réservoir de fluide extensible (23) vers le second tube (8) est établi par l'intermédiaire des cratères (37) .

8. - Sphincter artificiel (1) selon la revendication 5, dans lequel le clapet de non-retour sensible à la pression (3) comporte une bille (33) située à l'intérieur d'une cavité (34), ladite bille permettant un écoulement de fluide de la lumière (28) vers le ballonnet de compensation de pression (24) uniquement pendant un gonflage du moyen d'occlusion (20).

9. - Sphincter artificiel (1) selon la revendication 5, dans lequel le clapet de non-retour sensible à la pression (3) comporte un siège perméable (32) qui est situé entre la bille (33) et la lumière (28) et qui garantit que la bille (33) permet toujours, en raison de sa géométrie sphérique, un passage d'écoulement de fluide du ballonnet de compensation de pression (24) vers la lumière (28) .

10. - Sphincter artificiel (1) selon la revendication 4, dans lequel la bille (33) a une densité qui est sensiblement égale à la densité du fluide de pressurisation de telle sorte que la bille flotte librement dans ladite cavité (34).

11. - Sphincter artificiel (1) selon la revendication 4, dans lequel le réservoir (23) est fait d'un matériau élastique qui peut s'étendre par étirement de telle sorte que, dans l'état étiré, le fluide de pressurisation contenu dans ledit réservoir (23) a une pression supérieure à la pression abdominale du patient en raison de l'étirement du matériau élastique.

12. - Sphincter artificiel (1) selon la revendication 1, dans lequel le moyen d'occlusion (20) est dimensionné et formé pour occlure un canal anal ou urétral d'un être humain.
